# EUROPÄISCHE PATENTANMELDUNG

(11) **EP 0 556 726 A1**
(43) Veröffentlichungstag der Anmeldung: **25.08.1993**
(21) Anmeldenummer: 93102175.2
(22) Anmeldetag: 11.02.1993
(51) Int. Cl.: C07K 3/00, C07K 3/08, C12N 9/96

(54) **Verfahren zur Stabilisierung von Proteinen**

(30) Priorität: 13.02.1992 DE 4204313
(71) Anmelder: BOEHRINGER MANNHEIM GMBH, D-68298 Mannheim (DE)
(72) Erfinder: Buchner, Johannes, Dr., W-8400 Regensburg (DE); Madeker, Judith, W-8400 Regensburg (DE); Rudolph, Rainer, Prof. Dr., W-8120 Weilheim (DE)
(74) Vertreter: Huber, Bernhard, Dipl.-Chem.

(57) **Zusammenfassung**

Die vorliegende Erfindung betrifft ein Verfahren zur Stabilisierung von Proteinen in einer wäßrigen Lösung, welches dadurch gekennzeichnet ist, daß man der wäßrigen proteinhaltigen Lösung ein oder mehrere Vertreter aus der DnaJ-Proteinfamilie zusetzt.

## Beschreibung

Die vorliegende Erfindung betrifft ein Verfahren zur Stabilisierung von Proteinen in einer wäßrigen Lösung durch Zusatz einer als Stabilisator wirkenden Proteinkomponente.

Hitzeschock- oder Streßproteine werden sowohl konstitutiv exprimiert als auch induziert durch Stressoren/Umweltreize stark überexprimiert. Viele Vertreter dieser Proteinfamilie sind für den jeweiligen Organismus essentiell. Es wird zunehmend deutlich, daß diese Proteine in vivo eine wichtige Funktion bei der Strukturbildung von Proteinen unter physiologischen und Streßbedingungen erfüllen.

Für die E.coli Hitzeschockproteine GroEL und GroES konnte gezeigt werden, daß sie in vitro die Renaturierung einer Reihe von chemisch denaturierten Proteinen unterstützen und so die Ausbeuten steigern. GroEL bindet dabei das nicht-native Protein und verhindert dessen Aggregation (Buchner, J., Schmidt, M., Fuchs, M., Jaenicke, R., Rudolph, R., Schmid, R.X. und Kiefhaber, T. (1991) Biochemistry 30: 1586-1591). Eine ähnliche Funktion scheint es auch unter Hitzeschockbedingungen, d.h. bei erhöhter Temperatur auszuüben. Es bindet dabei denaturierendes Protein und hindert es so an der irreversiblen Aggregation (Höll-Neugebauer, B., Rudolph, R., Schmidt, M. und Buchner, J. (1991) Biochemistry 30: 11610-11614). Für DnaK, ein weiteres Hitzeschockprotein aus E.coli, wurde gezeigt, daß es durch seine Anwesenheit im hohen molaren Überschuß (60fach) während der thermischen Inaktivierung eines Proteins dessen irreversible Aggregation ATP-abhängig verhindern kann. Darüber hinaus scheinen auch bereits gebildete Aggregate von DnaK ATP-abhängig wieder aufgelöst werden zu können (Skowyra, D., Georgopoulos, C. und Zylicz, M. (1990) Cell 62: 939-944).

DnaJ ist ein weiteres Hitzeschockprotein aus E.coli. Es wird unter physiologischen Bedingungen exprimiert. Unter Hitzeschockbedingungen steigt sein Anteil am zellulären Protein um das Zehnfache an (Bardwell, J.C.A., Tilly, K., Craig, E., King, J., Zylicz, M. und Georgopoulos, C. (1986) J.Biol.Chem. 261: 1782-1785).

Mutationen in DnaJ führen zu einer Temperatursensitivität des Bakterienwachstums und zu Defekten in der DNA und RNA Synthese (Wada, M., Kadokami, Y. und Itikawa, H. (1982) Jpn. J. Genet. 57: 407-413; Itikawa, H. und Ryu, J. (1979) J. Bacteriol. 138: 339-344).

DnaJ besitzt eine Molekularmasse von 37 kDa unter denaturierenden bzw. 76 kDa unter nativen Bedingungen. Demzufolge liegt DnaJ in der aktiven Form als Dimer vor (Zylicz, M., Yamamoto, T., McKittrick, N., Sell, S. und Georgopoulos, C. (1985) J.Biol.Chem. 260: 7591-7598).

Entdeckt wurde DnaJ als essentielles Wirtsprotein für die Replikation des Bakteriophagen λ (Sunshine, M., Feiss, M., Stuart, J. und Yochem, J. (1977) Mol.Genet. 151: 27-34; Saito, H. und Udida, H. (1977) J.Mol.Biol. 113: 1-25; Zylicz, M., Yamamoto, T., McKittrick, N., Sell, S. und Georgopoulos, C. (1985) J.Biol.Chem. 260: 7591-7598). Es konnte gezeigt werden, daß DnaJ in dieser Reaktion mit DnaK zusammenwirkt (Liberek, U., Georgopoulos, C. und Zylicz, M. (1988) Proc. Natl.Acad.Sci. USA 85: 6632-6636). Bekannt ist auch, daß DnaK und DnaJ zusammen mit GrpE an der Replikation von Plasmiden beteiligt sind (Wickner, S., Hoskins, J. und McKenney, K. (1991) Nature 350: 165-167). DnaJ bewirkt eine zweifache Steigerung ATPase-Aktivität von DnaK; wird zusätzlich zu DnaJ auch noch das E.coli Hitzeschockprotein GrpE eingesetzt, so beobachtete man eine fünfzigfache Steigerung der ATPase-Aktivität (Liberek, M., Marszalek, J., Ang, D., Georgopoulos, C. und Zylicz, M. (1991) Proc.Natl.Acad.Sci. USA 88: 2874-2878).

Dabei beschleunigt DnaJ die ATP Hydrolyse, während GrpE die Freisetzung von ADP, das an DnaK gebunden ist, katalysiert.

In den letzten Jahren wurden mehrere zu DnaJ homologe Proteine in Eukaryonten identifiziert. In Hefe waren diese SIS1 (Luke, M.M., Sutton, A. und Arndt, K.T. (1991) J.Cell Biol. 114: 623-638), YDJ1 (Caplan, A.J. und Douglas, M.G. (1991) J.Cell Biol. 114: 609-621; Atencio et al., Mol.Cell Biol. 12 (1992), 283-291), Sec63 (Rothblatt et al., J.Cell Biol. 109 (1989), 2641-2652 und Sadler et al., J.Cell Biol. (1989), 2665-2675) und SCJ1 (Blumberg, H. und Silver, P. (191) Nature 349: 327-330). Im Menschen wurde das zu DnaJ homologe Protein als HDJ1 bezeichnet (Raabe, T. und Manley, J.L., Nucleic Acids Research (1991) 19: 6645). DnaJ und diese homologen Proteine werden nachstehend als "DnaJ Proteinfamilie" bezeichnet.

Eine Stabilisierung von Proteinen durch Zusatz von unterschiedlichen als Stabilisator wirkenden Proteinkomponenten ist bekannt. Die Nachteile bekannter Stabilisatorproteine bestehen jedoch darin, daß sie nur in Gegenwart von ATP wirksam sind, in großem molekularem Überschuß eingesetzt werden müssen oder/und selbst komplizierte, aus über 10 Untereinheiten zusammengesetzte Oligomere darstellen.

Aufgabe der Erfindung ist es somit, ein neues Verfahren zur Stabilisierung von Proteinen in einer wäßrigen Lösung bereitzustellen, bei dem diese oben genannten Nachteile mindestens teilweise beseitigt sind.

Die erfindungsgemäße Aufgabe wird durch die Bereitstellung eines Verfahrens zur Stabilisierung von Proteinen in einer wäßrigen Lösung gelöst, welches dadurch gekennzeichnet ist, daß man der wäßrigen proteinhaltigen Lösung einen oder mehrere Vertreter aus der DnaJ-Proteinfamilie Zusetzt.

Es wurde überraschenderweise festgestellt, daß DnaJ-Proteine andere Proteine in einer wäßrigen Lösung stabilisieren können, wobei diese stabilisierende Wirkung insbesondere auf einer Verhinderung der Bildung von unlöslichen Aggregaten beruht. Das erfindungsgemäße Verfahren kann beispielsweise zur Unterstützung bei der in vitro Faltung von denaturierten Proteinen, aber auch zur Stabilisierung von nativen Proteinen in gelöstem Zustand verwendet werden.

Das erfindungsgemäße Verfahren kann bei beliebigen Proteinen angewandt werden. Vorzugsweise wird es bei solchen Proteinen verwendet, die wegen ihrer Temperaturempfindlichkeit oder aus sonstigen Gründen in gelöstem Zustand leicht zur Aggregation neigen. Ein wichtiges Anwendungsgebiet für das erfindungsgemäße Verfahren ist die Stabilisierung von Lösungen, die enzymatisch aktive Proteine (z.B. Citratsynthase oder α-Glucosidase) enthalten.

Die DnaJ-Proteine bewirken eine Stabilisierung von Proteinen bei an sich beliebigen Proteinkonzentrationen, wobei jedoch eine besonderes gute Stabilisierungswirkung in einem Konzentrationsbereich von 0,0001 µmol/l bis 100 µmol/l des zu stabilisierenden Proteins erzielt wird.

Das molare Verhältnis zwischen dem zu stabilisierenden Protein und dem zugesetzten DnaJ-Protein beträgt bei dem erfindungsgemäßen Verfahren vorzugsweise 0,0001 : 1 bis 10 : 1. Dieses molare Verhältnis bezieht sich dabei auf den im allgemeinen zwei Untereinheiten aufweisenden DnaJ-Komplex. Besonders bevorzugt wird das DnaJ-Protein in einem molaren Verhältnis von 0,0001 : 1 bis 5 : 1 bezüglich der zu stabilisierenden Proteine zugesetzt. Dies bedeutet, daß DnaJ auch im substöchiometrischen Bereich die unerwünschte Aggregationsbildung unterbindet.

Ein besonderer Vorteil des erfindungsgemäßen Verfahrens besteht darin, daß die Stabilisierung der Proteine in Abwesenheit von ATP erfolgen kann, bzw. daß auch das Vorhandensein von ATP oder anderen Nukleosidtriphosphaten im allgemeinen keinen Einfluß auf die Stabilisierungswirkung des DnaJ-Proteins zeigt. Auf diese Weise ist es ohne weiteres möglich, Proteinlösungen für enzymatische Tests zu stabilisieren, bei denen ATP als Cosubstrat eingesetzt wird, ohne daß dadurch ein störender Einfluß auf den enzymatischen Test auftritt.

Die erfindungsgemäße Stabilisierung von Proteinen wird mit allen bekannten Proteinen der DnaJ-Proteinfamilie beobachtet, ob sie aus prokaryontischen oder eukaryontischen Organismen stammen. Die Wirksamkeit von DnaJ-Proteinen im Rahmen der Erfindung kann durch Zusatz von GrpE-Protein oder/und DnaK-Protein noch unterstützt werden. Die Reinigung von DnaJ erfolgt beispielsweise nach dem Zylicz et al. beschriebenen Verfahren (Zylicz, M., Gorska, J., Tyler, K., Georgopoulos, C. (1984) Mol.Gen.Genet. 196, 401-406).

Eine bevorzugte Anwendung des erfindungsgemäßen Verfahrens besteht in der Unterstützung bei einer in vitro Faltung bzw. Renaturierung von denaturierten Proteinen. Dabei handelt es sich vorzugsweise um Proteine, die durch heterologe Expression in Prokaryonten synthetisiert worden sind und dabei im allgemeinen in Form von Inclusion Bodies anfallen. Vorzugsweise handelt es sich bei den heterolog exprimierten Proteinen um eukaryontische Proteine. Der Begriff "heterologe Expression" bedeutet in diesem Zusammenhang, daß ein Protein aus einem fremden (heterologen) Organismus oder/und unter Kontrolle eines fremden (heterologen) Promotors in einer Zelle, insbesondere einer prokaryontischen Zelle exprimiert und anschließend aus der Zelle oder dem Kulturmedium gewonnen wird. Diese heterologe Expression von Proteinen ist ein dem Fachmann auf dem Gebiet der Molekularbiologie bekanntes Standardverfahren, auf das hier nicht näher eingegangen werden muß. In diesem Zusammenhang wird z.B. auf die Darstellung der Expression klonierter Gene bei Sambrook et al., (Molecular Cloning. A Laboratory Manual, Cold Spring Harbor Laboratory Press (1989), Kapitel 16 und 17) verwiesen.

Ein weiteres bevorzugtes Anwendungsgebiet des erfindungsgemäßen Verfahrens ist die Stabilisierung von nativen Proteinen in gelöstem Zustand. So kann beispielsweise durch Zusatz von DnaJ-Proteinen eine Konstanthaltung der Empfindlichkeit bei optischen Tests in proteinhaltigen Lösungen erreicht werden, bei denen Störungen durch eine geringe Stabilität von in der Testlösung vorhandenen Proteinkomponenten auftreten können. Ein derartiger optischer Test beinhaltet vorzugsweise eine enzymatische Reaktion, besonders bevorzugt eine enzymatische Reaktion, bei der ATP als Cosubstrat vorhanden ist.

Ein "optischer Test" im Sinne der vorliegenden Erfindung ist ein Bestimmungsverfahren, bei dem eine optische Größe oder die Änderung einer optischen Größe, z.B. Absorption, Transmission, Streulicht etc. gemessen wird. Vorzugsweise wird die vorliegende Erfindung bei Verfahren angewendet, in denen das Streulicht bestimmt wird, z.B. bei turbidometrischen, nephelometrischen oder fluorimetrischen Verfahren.

Weiterhin können auch proteinhaltige Lösungen stabilisiert werden, die Antikörper oder von Antikörpern abgeleitete Derivate enthalten.

Ein weiterer Gegenstand der vorliegenden Erfindung ist auch ein Reagenz zur Stabilisierung von Proteinen in wäßriger Lösung, das fest oder flüssig sein kann und ein oder mehrere DnaJ-Proteine, insbesondere das DnaJ-Protein aus E.coli enthält. Ein erfindungsgemäßes Reagenz kann beispielsweise das DnaJ-Protein in gelöster Form oder/und als Lyophilisat enthalten.

Die Erfindung soll weiterhin durch die folgenden Beispiele in Verbindung mit der Zeichnung erläutert werden. Es zeigt:
- Abb. 1: den Aggregationsverlauf bei einer Renaturierung von Citratsynthase in Abhängigkeit von der DnaJ-Konzentration,
- Abb. 2: den Aggregationsverlauf bei einer Renaturierung von α-Glucosidase in Gegenwart und Abwesenheit von DnaJ.

### Beispiel 1

### Einfluß von DnaJ auf die Aggregation renaturierender Citratsynthase

Es wurde eine Denaturierung von Citratsynthase durch Inkubation von 15 µmol/l Citratsynthase in 6,0 mol/l Guanidin·HCl (Gdn·HCl), 20 mmol/l DTE, 50 mmol/l Tris/HCl, 2 mmol/l EDTA, pH 8,0 (Puffer A) für 2 h bei 20°C durchgeführt.

Anschließend erfolgte die Renaturierung von Citratsynthase in Abwesenheit bzw. in Gegenwart von DnaJ. Dazu wurde denaturierte Citratsynthase (s.o.) unter Rühren in einem Verhältnis von 1:100 in 10 mmol/l MOPS, 50 mmol/l KCl, pH 7,6 (Puffer B) mit unterschiedlichen DnaJ-Konzentrationen verdünnt. Die Aggregation von Citratsynthase (Endkonzentration 0,15 µmol/l) wurde durch Messung der Lichtstreuung bei einer Emissions- und Excitationswellenlänge von 500 nm über die ersten 5 Minuten erfaßt. Die Totzeit beträgt etwa 5 Sekunden. Die Aggregation ist in willkürlichen Einheiten angegeben. Das Gesamtvolumen des Reaktionsansatzes beträgt 2 ml.

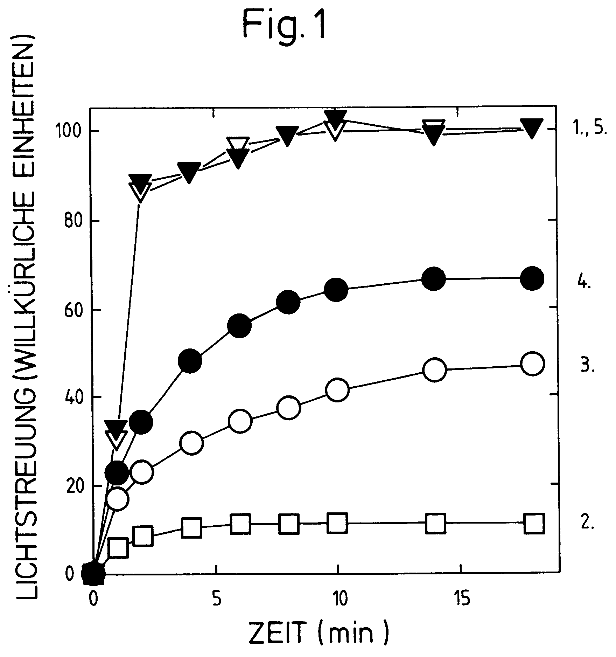

Es wurden insgesamt 5 Reaktionsansätze durchgeführt
1. Puffer B
2. Puffer B, 0,15 µmol/l DnaJ
3. Puffer B, 0,023 µmol/l DnaJ
4. Puffer B, 0,0045 µmol/l DnaJ
5. Puffer B, 0,7·10⁻³ µmol/l DnaJ
Die Ergebnisse zeigt Figur 1 der Zeichnung.

### Beispiel 2

### Einfluß von DnaJ auf die Aggregation renaturierender α-Glucosidase

α-Glucosidase PI wurde in Hefe (Stamm ABYSMAL81) exprimiert (Kopetzki et al. (1989), EP 0 323 838, Kopetzki et al., Yeast 5 (1989), 11-24) und mit den üblichen Methoden der Ionenaustausch- und hydrophoben Interaktionschromatographie gereinigt.

### Denaturierung der α-Glucosidase:

Inkubation von 14,7 µmol/l α-Glucosidase in 10 mmol/l Kaliumphosphat, 1 mmol/l EDTA, 2 mmol/l DTE, 8 mol/l Harnstoff, pH 7,0 für 1 h bei Raumtemperatur (23°C).

### Renaturierung der α-Glucosidase:

Denaturierte α-Glucosidase wird unter Rühren 1:100 in Kaliumphosphatpuffer +/- DnaJ verdünnt (40 mmol/l Kaliumphosphat, 0,1 mmol/l EDTA, 0,1 mol/l KCl, 5 mmol/l DTE, pH 6,8). Die Aggregation der α-Glucosidase wird durch Messung der Lichtstreuung im Fluoreszenzspectrophotometer bei Ex.360 nm/Em.360nm verfolgt.

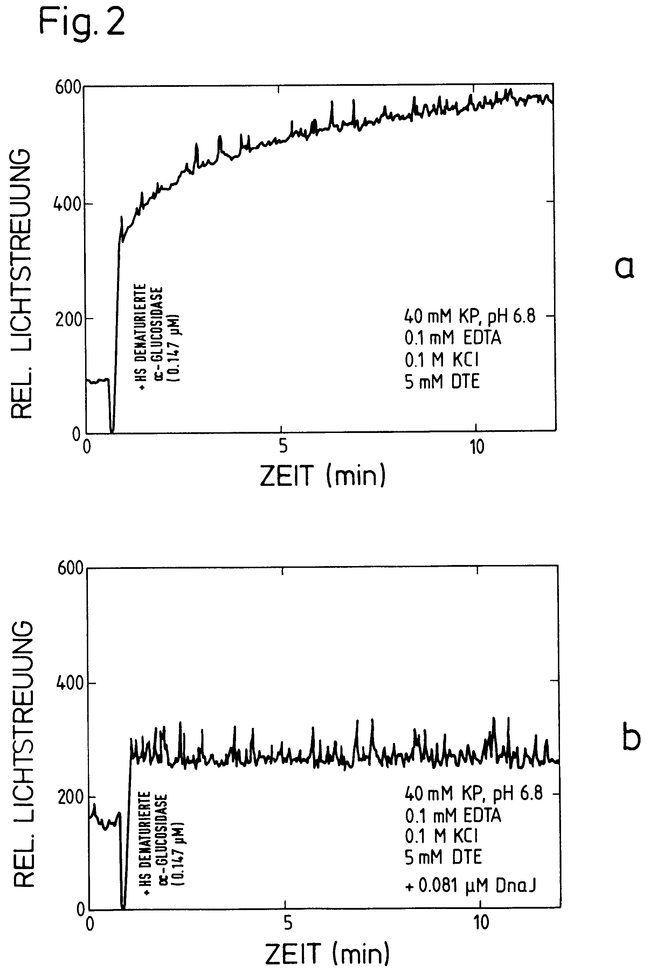

Die Ergebnisse zeigt Figur 2 a und b der Zeichnung im Vergleich der durch die Aggregation hervorgerufenen Lichtstreuung ohne (a) und mit (b) DnaJ.

## Patentansprüche

1. Verfahren zur Stabilisierung von Proteinen in einer wäßrigen Lösung,
**dadurch gekennzeichnet,**
daß man der wäßrigen proteinhaltigen Lösung ein oder mehrere Vertreter aus der DnaJ-Proteinfamilie zusetzt.

2. Verfahren nach Anspruch 1,
**dadurch gekennzeichnet,**
daß man das DnaJ-Protein in einem molaren Verhältnis von 0,0001 : 1 bis 10 : 1 bezüglich der zu stabilisierenden Proteine zusetzt.

3. Verfahren nach Anspruch 1 oder 2,
**dadurch gekennzeichnet,**
daß das zu stabilisierende Protein ein enzymatisch aktives Protein ist.

4. Verfahren nach einem der Ansprüche 1 bis 3,
**dadurch gekennzeichnet,**
daß man DnaJ aus E.coli verwendet.

5. Verfahren nach einem der Ansprüche 1 bis 4,
**dadurch gekennzeichnet,**
daß man auch ein GrpE-Protein oder/und DnaK-Protein zusetzt.

6. Verwendung von DnaJ-Proteinen zur Unterstützung bei der in vitro-Renaturierung von denaturierten Proteinen,
**dadurch gekennzeichnet,**
daß es sich bei dem denaturierten Protein um ein Protein handelt, das durch heterologe Expression in Prokaryonten synthetisiert worden ist.

7. Verwendung von DnaJ-Proteinen zur Stabilisierung von nativen Proteinen in gelöstem Zustand.

8. Verwendung nach Anspruch 7 zur Konstanthaltung der Empfindlichkeit bei optischen Tests in proteinhaltigen Lösungen, bei denen Störungen durch eine geringe Stabilität von in der Testlösung vorhandenen Proteinkomponenten auftreten können.

9. Verwendung von DnaJ nach einem der Ansprüche 6 bis 8 in Kombination mit einem GrpE- oder/und DnaK-Protein.

10. Reagenz zur Stabilisierung von Proteinen in wäßriger Lösung,
**dadurch gekennzeichnet,**
daß es ein oder mehrere DnaJ-Proteine enthält.

11. Reagenz nach Anspruch 10,
**dadurch gekennzeichnet,**
daß das DnaJ-Protein aus E.coli stammt.

12. Reagenz nach einem der Ansprüche 16 bis 18,
**dadurch gekennzeichnet,**
daß es auch ein oder mehrere GrpE- oder/und DnaK-Proteine enthält.
